(19) Europäisches Patentamt / European Patent Office / Office européen des brevets

(11) **EP 3 569 143 B1**

(12) **EUROPÄISCHE PATENTSCHRIFT**

(45) Veröffentlichungstag und Bekanntmachung des Hinweises auf die Patenterteilung:
**06.11.2024   Patentblatt 2024/45**

(21) Anmeldenummer: **18172315.6**

(22) Anmeldetag: **15.05.2018**

(51) Internationale Patentklassifikation (IPC):
**A61B 5/276** (2021.01)   **A61B 5/30** (2021.01)
A61B 5/308 (2021.01)   A61B 5/31 (2021.01)
A61B 5/313 (2021.01)

(52) Gemeinsame Patentklassifikation (CPC):
**A61B 5/7221; A61B 5/276; A61B 5/30;**
**A61B 5/7225;** A61B 5/308; A61B 5/31; A61B 5/313;
A61B 2562/222

(54) **DETEKTION VON SIGNALPFADDEFEKTEN BEI DER MESSUNG VON BIOELEKTRISCHEN SIGNALEN**

DETECTION OF SIGNAL PATH EFFECTS IN THE MEASUREMENT OF BIOELECTRIC SIGNALS

DÉTECTION DE DÉFAILLANCES DE VOIE DE SIGNAL LORS DE LA MESURE DE SIGNAUX BIOÉLECTRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.11.2019   Patentblatt 2019/47**

(73) Patentinhaber: **Siemens Healthineers AG**
**91301 Forchheim (DE)**

(72) Erfinder: **Batzer, Ulrich**
**91054 Buckenhof (DE)**

(74) Vertreter: **Siemens Healthineers**
**Patent Attorneys**
**Postfach 22 16 34**
**80506 München (DE)**

(56) Entgegenhaltungen:
EP-B1- 0 944 413          DE-A1- 19 728 902
US-A- 5 766 133          US-A1- 2003 163 170
US-A1- 2017 071 548

**Beschreibung**

Die Erfindung betrifft eine Fehlerdetektionsvorrichtung sowie ein Verfahren zur Detektion von Signalpfaddefekten in einem Spannungsmesssystem, vorzugsweise einem differentiellem Spannungsmesssystem, mit einer Signalmessschaltung zur Messung von bioelektrischen Signalen mit einer Anzahl von Nutzsignalpfaden. Zudem betrifft die Erfindung ein Spannungsmesssystem mit einer solchen Fehlerdetektionsvorrichtung.

Spannungsmesssysteme, insbesondere differentielle Spannungsmesssysteme, zur Messung von bioelektrischen Signalen werden beispielsweise in der Medizin zur Messung von Elektrokardiogrammen (EKG), Elektroenzephalogrammen (EEG) oder Elektromyogrammen (EMG) genutzt. Bei solchen Anwendungen sollte vorzugsweise auf jedem Messkanal auf eine hohe Eingangsimpedanz von mindestens mehreren MOhm geachtet werden, um den Einfluss von Störungen zu reduzieren oder zumindest nicht zu verstärken. Die hohe angestrebte Eingangsimpedanz sollte auch in den Kabeln der oben genannten Geräte zur Messung von bioelektrischen Signalen gehalten werden. Üblicherweise sind die Messleitungen der Kabel von einer Schirmung umgeben. Um eine bessere Handhabbarkeit zu erlangen, sind die Kabel zudem flexibel, schlank und leicht. Diese Merkmale führen jedoch zu einem Konflikt zwischen der Lebensdauer und der Handhabbarkeit der Kabel und erhöhen das Risiko eines Kabeldefekts.

Die Ursache für defekte Kabel sind beispielsweise gebrochene Leitungen oder oftmals Kinken. Kinken können nach einer Torsion oder Beugung der langen Kabel entstehen, wenn die Messleitungen sich auswölben und nicht in ihre ursprüngliche Form zurückkehren. Die ausgewölbten Messleitungen können dann die Leitungs-Isolation durchbrechen und die Schirmung kontaktieren, was zu einer verringerten Eingangsimpedanz in den Kabeln und damit zu einer Verstärkung von Störungen führen kann. Das Problem hierbei ist, dass sich solche Störungen ohne spezielle Testumgebung nicht immer eindeutig auf eine verminderte Eingangsimpedanz in den Kabeln zurückführen lassen. Zudem treten Kinken und andere Eingangsimpedanz-reduzierende Kabeldefekte oftmals als Wackelkontakte auf, was eine Fehlerdetektion ebenfalls erschwert. Daher besteht die Gefahr, dass unbeabsichtigt mit einem defekten Kabel gearbeitet wird, bis die Signalqualität nicht mehr ausreichend ist, um bioelektrische Messungen und Untersuchungen durchführen zu können.

Eine gängige Lösung, um Störungen durch eine verminderte Eingangsimpedanz in den Kabeln zu vermeiden oder zu detektieren ist es, die Kabel in regelmäßigen Abständen auszutauschen oder zumindest durch Servicepersonal untersuchen zu lassen. Beim Überprüfen der Kabel durch Servicepersonal wird ein von einem Simulator erzeugtes Referenzsignal auf das zu untersuchende Kabel aufgegeben. Daraufhin wird das Kabel bewegt und ein Ausgangssignal wird gemessen. Dieses Ausgangssignal wird mit dem Referenzsignal verglichen und überprüft, ob es Abweichungen zwischen den beiden Signalen und somit Störungen gibt. Bei welchen Störungen ein Kabelbruch angenommen wird, beruht jedoch auf der Expertenmeinung. Zudem ist diese Überprüfung relativ aufwendig.

In US 5,776,133 werden Spannungsmessungen an Leitungskabeln eines EKG-Messsystems durchgeführt, um einen Kabelbruch zu detektieren.

Es ist daher eine Aufgabe der vorliegenden Erfindung, eine Vorrichtung sowie ein Verfahren zur Verfügung zu stellen, um Signalpfaddefekte, insbesondere Kabeldefekte, in einem Spannungsmesssystem, zu detektieren.

Diese Aufgabe wird durch eine Fehlerdetektionsvorrichtung gemäß Patentanspruch 1, durch ein Spannungsmesssystem gemäß Patentanspruch 11 sowie durch ein Verfahren gemäß Patentanspruch 13 gelöst.

Die erfindungsgemäße Fehlerdetektionsvorrichtung detektiert in einem eingangs genannten Spannungsmesssystem Signalpfaddefekte, insbesondere Kabeldefekte der Kabel, die z.B. vom Patienten zu einem Messgerät des differentiellen Spannungsmesssystems führen. Dieses Spannungsmesssystem, vorzugsweise ein differentielles Spannungsmesssystem, beispielsweise ein EKG-Messsystem, ein EEG-Messsystem oder ein EMG-Messsystem, weist wie eingangs erläutert, eine Signalmessschaltung mit einer Anzahl von Nutzsignalpfaden bzw. Messleitungen zur Messung der bioelektrischen Signale auf. Erfindungsgemäß weist die Fehlerdetektionsvorrichtung zumindest eine erste Stromaufbringungseinheit auf.

Dabei ist die Stromaufbringungseinheit so ausgebildet, dass sie vorzugsweise ein beliebiges aber definiertes Signal auf einen ersten Nutzsignalpfad der Signalmessschaltung des Spannungsmesssystems aufprägt. Bei dem Signal kann es sich vorzugsweise um einen Strom handeln, der direkt oder indirekt aufgeprägt und gemessen werden kann. So kann der Strom bevorzugt über eine Stromquelle auf einen Nutzsignalpfad aufgeprägt werden. Der Strom kann aber auch besonders bevorzugt über einen Pull-up-Widerstand und/oder einen Pull-up-down-Widerstand indirekt auf dem Nutzsignalpad aufgeprägt bzw. geregelt werden. Der aufgeprägte Strom liegt dabei im Nanoampere-Bereich, um gemessene bioelektrische Signale nicht zu verfälschen und damit eine Gefährdung eines Patienten ausgeschlossen wird.

Um das Signal zu detektieren bzw. zu messen weist die erfindungsgemäße Fehlerdetektionsvorrichtung zumindest eine erste Vergleichseinheit auf, welche überprüft, ob das aufgeprägte Signal des ersten Nutzsignalpfads innerhalb eines Messbereichs liegt, bei dem davon ausgegangen wird, dass das Signal auf dem Nutzsignalpfad fließt. Hierzu wird ein definierter Messbereich gewählt, ab dem davon ausgegangen wird, dass das Signal auf dem Nutzsignalpfad fließt.

Eine weitere Komponente der Fehlerdetektionsvorrichtung ist zumindest ein als Strommesspfad ausgebildeter erster Störsignalpfad zur stromerfassenden Messung eines ersten Störsignals. Bei der Messung von bioelektrischen Signalen, beispielsweise von EKG-Signalen, treten bekanntermaßen häufig Gleichtaktstörsignale, auch "Common Mode"-Signale (CM-Signale) genannt, auf. Sie ergeben sich z. B. aus der Netzfrequenz mit 50Hz.

Im Regelfall, also bei intakten Nutzsignalpfaden bzw. Kabeln, sind die Eingangsimpedanzen der Messleitungen der Kabel eines EKG-Messsystems so hoch, dass das Störsignal, das auf dem ersten Störsignalpfad gemessen wird diesen Gleichtaktstörsignalen entspricht.

Die Fehlerdetektionsvorrichtung umfasst außerdem eine Signalpfaddefektanalyseeinheit, welche ausgebildet ist, um einen Signalpfaddefekt in einem Nutzsignalpfad des Spannungsmesssystems zu detektieren. Dieser Signalpfaddefekt wird hier erfindungsgemäß detektiert, wenn das aufgeprägte Signal nicht auf dem ersten Störsignalpfad gemessen wird und das überprüfte Signal der Vergleichseinheit innerhalb des Messbereichs liegt. Die Signalpfaddefektanalyseeinheit kann dabei unterschiedlich ausgebildet sein. Sie kann bevorzugt einen Integrated Circuit umfassen, besonders bevorzugt einen ASIC. Die Signalpfaddefektanalyseeinheit kann aber auch vorzugsweise einen Mikrocontroller umfassen oder eine sonstige universelle Recheneinheit.

Bei dem Verfahren zur Detektion von Signalpfaddefekten in dem zuvor erläuterten Spannungsmesssystem, wird dementsprechend erfindungsgemäß ein erstes Signal auf einen ersten Nutzsignalpfad mittels einer Stromaufbringungseinheit aufgeprägt, derart, dass der aufgeprägte Strom im Nanoampere-Bereich liegt, um gemessene bioelektrische Signale nicht zu verfälschen.

Daraufhin erfolgt eine Überprüfung, mittels zumindest einer ersten Vergleichseinheit, ob das Signal, das auf den ersten Nutzsignalpfad aufgeprägt wurde, innerhalb eines Messbereichs liegt, bei dem davon ausgegangen wird, dass das Signal auf dem Nutzsignalpfad fließt. Zeitgleich oder aber auch danach wird zumindest stromerfassend ein erstes Störsignal auf zumindest einem als Strommesspfad ausgebildeten ersten Störsignalpfad gemessen. Daraufhin findet eine Analyse mittels einer Signalpfaddefektanalyseeinheit statt, um einen Signalpfaddefekt in einem Nutzsignalpfad des Spannungsmesssystems zu detektieren, wenn das aufgeprägte Signal nicht auf dem ersten Störsignalpfad gemessen wird und das überprüfte Signal der Vergleichseinheit innerhalb des Messbereichs liegt.

Ist der Nutzsignalpfad beispielsweise mittels einer Elektrode mit einem Patienten verbunden und prägt die Stromaufbringungseinheit ein Signal, bzw. einen Strom auf den ersten Nutzsignalpfad auf, so fließt dieser Strom über den Patienten und einen entsprechend ausgelegten Rückpfad, der mit einem Patienten verbunden ist wieder ab.

Bei einem entsprechend ausgelegten Rückpfad für den auf dem Nutzsignalpfad aufgeprägten Strom handelt es sich vorzugsweise um einen niederohmigen Rückpfad auf ein gemeinsames Bezugspotential. Solch ein Rückpfad wird beispielsweise durch den Störpfad gebildet. Da die Nutzsignalpfade aufgrund ihrer hohen Eingangsimpedanzen keinen niederohmigen Rückflusspfad auf das gemeinsame Bezugspotential bilden (bei intakten Kabeln), kann das aufgeprägte Signal nur über den Störsignalpfad abfließen. Das hat zur Folge, dass sich nicht nur die oben beschriebenen Störsignale auf dem Störsignalpfad befinden, sondern auch das auf dem Nutzsignalpfad aufgeprägte Signal.

Ist der erste Nutzsignalpfad nicht mit dem Patienten verbunden, da sich beispielsweise die Elektrode vom Patienten gelöst hat, so ist der Stromkreis nicht geschlossen bzw. die vom aufgeprägten Signal zu überwindende Impedanz ist wesentlich größer.

Daher geht die Spannung, welche durch die Stromaufbringungseinheit beispielsweise an der Elektrode des ersten Nutzsignalpfads bewirkt wurde, in die Sättigung. Der Strom kann dabei beispielsweise indirekt über einen Widerstand als Spannung detektiert werden. Das aufgeprägte Signal liegt somit außerhalb eines Messbereichs.

Diese Überprüfung bzw. Messung wird mittels der Vergleichseinheit durchgeführt. Die Vergleichseinheit kann also überprüfen, ob der Nutzsignalpfad mit einem Patienten verbunden ist oder nicht. Sie allein gibt aber keinen Aufschluss darüber, ob ein Signalpfaddefekt eines mit einem Patienten verbundenen Nutzsignalpfads vorliegt.

Ist ein Signalmesskabel bzw. Kabel eines Nutzsignalpfads defekt, so weist die Signalmesseschaltung neben dem Störsignalpfad mindestens einen weiteren niederohmigen Rückpfad für den aufgeprägten Strom auf. Das aufgeprägte Signal geht dann über in die Schirmung bzw. in Erde und fließt nicht mehr über den Störsignalpfad ab. Das Signal auf dem Störsignalpfad wird also bei einem Kabeldefekt nicht mehr um das auf dem Nutzsignalpfad aufgeprägte Signal erhöht.

Kann das aufgeprägte Signal nicht auf dem Störsignalpfad detektiert werden, die Vergleichseinheit gibt aber an, dass der Nutzsignalpfad, auf den das Signal aufgeprägt wurde, mit einem Patienten verbunden ist, so liegt ein Signalpfaddefekt des Nutzsignalpfads, auf den das Signal aufgeprägt wurde, vor. Diese Analyse wird mittels der Signalpfaddefektanalyseeinheit durchgeführt.

Die erfindungsgemäße Vorrichtung, gibt also zum einen Aufschluss darüber, ob ein Nutzsignalpfad mit einem Patienten verbunden ist und ob ein Signalpfaddefekt eines solchen Nutzsignalpfads vorliegt.

Im Gegensatz zum Stand der Technik, bei dem nur die Nutzsignalpfade bzw. die zu prüfenden Kabel selber geprüft werden, wird also erfindungsgemäß zusätzlich auf einem Störsignalpfad gemessen und überprüft, ob die auf die Nutzsignalpfade zusätzlich aufgeprägten Signale auf den Störsignalpfad übergekoppelt haben.

Die Nutzsignalpfade weisen hier also eine Doppelfunktion auf. Sie dienen zum einen dazu, bioelektrische Signale zu messen, wodurch sie einen Teil der Signalmessschaltung darstellen. Zum anderen werden auf die Nutzsignalpfade Ströme aufgeprägt, um zu detektieren, ob diese auf einen Störsignalpfad überkoppeln, wodurch die Nutzsignalpfade auch einen Teil der Fehlerdetektionsvorrichtung darstellen.

Ein Vorteil der hier beschriebenen erfindungsgemäßen Vorrichtungen und Verfahren ist, dass kein Techniker beziehungsweise Servicepersonal mehr benötigt wird, um einen Kabeldefekt zu detektieren. Die Messung auf dem Störsignalpfad und auf dem Nutzsignalpfad kann während einer Nutzsignalmessung parallel und automatisch verlaufen und ein Kabeldefekt kann sofort sichtbar gemacht werden, beispielsweise auf einer Benutzerschnittstelle des Spannungsmesssystems. Daher kann der Signalpfaddefekt, beziehungsweise Kabeldefekt, sofort z. B. durch das Bedienpersonal selber entdeckt werden und das Kabel kann sofort getauscht und eine korrekte Messung durchgeführt werden. Dadurch sinkt also auch die Gefahr, dass mit unentdeckt beschädigten Kabeln weiter gemessen wird.

Zudem spielt das Nutzsignal bei der Detektion eines Signalpfaddefekts keine Rolle. Daher kann die erfindungsgemäße Fehlerdetektionsvorrichtung für verschiedenste Spannungsmesssysteme, wie beispielsweise EKG-Messsysteme, EEG- Messsysteme oder EMG- Messsysteme Anwendung finden, ohne dafür speziell angepasst werden zu müssen. Hierdurch können ebenfalls enorme Entwicklungs- und Herstellungskosten eingespart werden.

Zudem benötigt die Fehlerdetektionsvorrichtung keine externe Spannungsquelle für eine Detektion eines Signalpfaddefekts, da alle relevanten stromführenden Teile in der Fehlerdetektionsvorrichtung bzw. der Spannungsmesseinrichtung integriert sind. Dadurch kann der Testaufbau komplett passiv sein.

Die erfindungsgemäße Fehlerdetektionsvorrichtung kann eine eigenständige Komponente sein und beispielsweise als Nachrüstsatz in bereits bestehende EKGs, EEGs oder EMGs z. B. über Steckverbindungen, eingebaut bzw. vor- oder zwischengeschaltet werden, was später noch näher erläutert wird. Vorzugsweise ist die Fehlerdetektionsvorrichtung jedoch bereits fest in einem erfindungsgemäßen Spannungsmesssystem integriert.

Ein Großteil der zuvor genannten Komponenten der Spannungsmesseinrichtung, insbesondere die Signalpfaddefektanalyseeinheit, kann ganz oder teilweise in Form von Softwaremodulen in einem Prozessor eines entsprechenden Spannungsmesssystems realisiert werden. Eine weitgehend softwaremäßige Realisierung hat den Vorteil, dass auch schon bisher verwendete Spannungsmesssysteme auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Spannungsmesssystems ladbar ist, mit Programmabschnitten, um alle Schritte des erfindungsgemäßen Verfahrens auszuführen, wenn das Programm in dem Spannungsmesssystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Spannungsmesssystem und/oder zur Speicherung an oder in dem Spannungsmesssystem kann ein computerlesbares Medium, beispielsweise ein Memorystick, eine Festplatte oder ein sonstiger transportabler oder fest eingebauter Datenträger dienen, auf welchem die von einer Rechnereinheit des Spannungsmesssystem einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bei einem eingangs genannten Spannungsmesssystem kann die Signalmessschaltung in Abhängigkeit ihrer Anwendung eine beliebige Anzahl an Nutzsignalpfaden bzw. Signalmesskabeln aufweisen. In der Regel weist eine Signalmessschaltung, beispielsweise ein EKG-Messsystem, zumindest zwei Nutzsignalpfade auf. Die Nutzsignalpfade umfassen vorzugsweise Elektroden, welche an einen zu untersuchenden Patienten angelegt werden können, um ein dort anliegendes elektrisches Potential zu messen. Der Aufbau der Elektroden kann von der genauen Art der Messung abhängen, z.B. ob es sich um eine EKG-Messung, eine EEG-Messung oder eine EMG- Messung handelt, und davon, wo genau am Patienten das Potential gemessen werden soll. Geeignete Elektroden für verschiedene Einsatzzwecke sind dem Fachmann bekannt. Der Ausgang der Elektroden ist, vorzugsweise über die genannten Signalmesskabel, bevorzugt mit einer Verstärkerschaltung verbunden. Besonders bevorzugt sind die Elektroden mit einem Differenzverstärker elektrisch verbunden. Dieser bildet aus den an seinen Eingängen gemessenen und von den Elektroden erfassten Signalen eine Differenz und verstärkt diese.. Zudem weist die Signalmessschaltung eine Signalerfassungseinheit auf, die am Ausgang der Verstärkerschaltung geschaltet ist, um die verstärkten Signale oder z.B. die Potentiale zu erfassen und weiter zu nutzen und/oder aufzuzeichnen. Z. B. kann die Signalerfassungseinheit einen A/D-Wandler und weitere Komponenten aufweisen, um das digitale Signal weiter zu verarbeiten.

Bevorzugt weist die Signalmessschaltung des differentiellen Spannungsmesssystems einen dritten Nutzsignalpfad auf. Des Weiteren umfasst die Signalmessschaltung vorzugsweise eine Treiberschaltung, welche zwischen einem Strommesswiderstand und die Signalerfassungseinheit geschaltet ist. Die Treiberschaltung wird auch als "Right-Leg-Drive" (RLD) bezeichnet und ist für die Erzeugung eines Signals verantwortlich, welches auf die Durchschnitts-Gleichtaktspannung einzelner oder aller Signale geregelt wird. Die bereits oben erwähnten und gemessenen Gleichtaktstörsignale, können dadurch in den Nutzsignalpfaden eliminiert werden.

Der dritte Nutzsignalpfad (bzw. "Right-Leg-Drive-Pfad") sorgt für einen Potentialausgleich zwischen dem Patienten und dem differentiellem Spannungsmesssystem bzw. dem EKG-Messsystem. Dabei ist die Elektrode des dritten Nutzsignalpfads vorzugsweise auf dem rechten Bein des Patienten angebracht, worauf die Bezeichnung "Right-Leg-Drive" zurückzuführen ist. Grundsätzlich kann dieses dritte Potential aber auch an anderer Stelle am Patienten erfasst werden.

Auch der erste Störsignalpfad der erfindungsgemäßen Fehlerdetektionsvorrichtung ist vorzugsweise über eine Elektrode mit dem Patienten verbunden. Daher kann vorzugsweise der dritte Nutzsignalpfad ganz oder zumindest teilweise mit dem ersten Störsignalpfad, welcher nachfolgend näher erläutert wird, zusammenfallen bzw. diesem zumindest abschnittsweise entsprechen. Z. B. können für den dritten Nutzsignalpfad und den ersten Störsignalpfad dieselbe Elektrode und dasselbe Kabel verwendet werden. Es ist also dann nicht einmal erforderlich, dass das Bedienpersonal zusätzliche Elektroden für die erfindungsgemäße Signalpfadprüfung am Patienten anlegt oder sonstige spezielle Maßnahmen durchführt.

Der erste Störsignalpfad weist bevorzugt eine Strommesseinheit auf. Diese Strommesseinheit umfasst vorzugsweise einen Strommesswiderstand, bei welchem es sich bevorzugt um einen Shunt-Widerstand handelt, und eine zu diesem parallel geschaltete Spannungsmesseinrichtung.

Der Strommesswiderstand kann dabei zwischen die dritte Elektrode und die Treiberschaltung der Signalmessschaltung, d.h. den Right-Leg-Drive, geschaltet werden.

Es ist bevorzugt, dass der Shunt-Widerstand mindestens einen Widerstandswert von 10 kΩ und maximal einen Widerstandswert von 1000 kΩ aufweist.

Bei der Spannungsmesseinrichtung handelt es sich vorzugsweise auch um einen Differenzverstärker. Der Störsignalpfad weist am Ausgang der Spannungsmesseinrichtung eine Störsignalerfassungseinheit auf, um das gemessene Störsignal weiter verarbeiten zu können. Die Störsignalerfassungseinheit umfasst z. B. einen A/D-Wandler und eine Einheit, um das digitale Signal weiter zu verarbeiten.

Beispielsweise kann innerhalb des ersten Störsignals oder eines daraus resultierenden bzw. weiterverarbeitenden Signals, z. B. im Zeit- und/oder Frequenzbereich, nach typischen Merkmalen des bioelektrischen Signals gesucht werden, beispielsweise in einem EKG-Signal nach den typischen EKG-Zacken.

Das Störsignal auf dem ersten Störsignalpfad weist in der Regel nur sehr geringe Gleichstrom-Anteile auf. Wird das Signal auf dem Nutzsignalpfad als Gleichstrom aufgeprägt, so lässt sich dieser Anteil sehr leicht vom Wechselstrom unterscheiden.

Ist das Störsignal aber beispielsweise bereits mit starken, zeitlich nicht konstanten Gleichstromanteilen beaufschlagt, so kann es von Vorteil sein, das Signal als einen Wechselstrom auf den Nutzsignalpfad aufzuprägen.

Daher ist die Stromaufbringungseinheit bzw. Stromaufbringungskontrolleinheit vorzugsweise so ausgebildet, dass sie einen Wechselstrom und/oder einen Gleichstrom auf die Nutzsignalpfade aufprägen kann.

Vorzugsweise ist die Stromaufbringungseinheit so ausgebildet, dass die aufgeprägten Signale auf die Nutzsignalpfade positive Ströme umfassen. Dabei wird auf die Nutzsignalpfade der Signalmessschaltung jeweils ein positiver Strom aufgeprägt. Somit ergibt sich beispielsweise ein Gesamtstrom, der auf den Störsignalpfad übergekoppelt werden kann, von

$$I_g = I_P * N$$

$I_g$: Gesamtstrom
$I_P$: positiver Strom
N: Anzahl Nutzsignalpfade

Liegt kein Signalpfaddefekt vor und sind alle Nutzsignalpfade mit dem Patienten verbunden, so fließt also zusätzlich zum Störsignal auch der durch die Stromaufbringungseinheiten aufgeprägte Gesamtstrom über den ersten Störsignalpfad ab.

Weist die Signalmessschaltung mehrere Nutzsignalpfade auf, so kann es zu einem Sättigungseffekten kommen, wenn alle Nutzsignalpfade mit einem positiven Storm beaufschlagt werden.

Daher ist die Stromaufbringungseinheit vorzugsweise so ausgebildet, dass sie auf die Nutzsignalpfade unterschiedliche Signale aufprägen kann.

Dazu weist die Fehlerdetektionsvorrichtung ganz besonders bevorzugt je Nutzsignalpfad eine Stromaufbringungseinheit auf. Eine Stromaufbringungseinheit umfasst bevorzugt eine Stromquelle. Besonders bevorzugt und wie bereits erwähnt umfasst die Stromaufbringungseinheit aber einen Pull-up- bzw. Pulldown-Widerstand, welcher die Spannungen entlang der Nutzsignalpfade herauf bzw. hinunter regelt und somit indirekt die aufgeprägten Ströme auf dem jeweiligen Nutzsignalpfad beeinflusst.

Dabei ist die Stromaufbringungseinheit bevorzugt so ausgebildet, dass sie auf eine Anzahl von Nutzsignalpfaden einen positiven Strom und auf eine Anzahl von Nutzsignalpfaden einen negativen Strom aufgeprägt. Besonders bevorzugt entspricht die Anzahl an Nutzsignalpfaden, auf welche ein positiver Strom aufgeprägt wird, der Anzahl an Nutzsignalpfaden, auf welche ein negativer Strom aufgeprägt wird. Dadurch kann abwechselnd ein Nutzsignalpfad mit einem positiven Strom beaufschlagt werden und ein Nutzsignalpfad mit eine negativen Strom beaufschlagt werden. Dadurch ergibt sich ein aufgeprägter Gesamtstrom von:

$$I_g = \frac{N}{2} * I_P - \frac{N}{2} * I_N$$

$I_N$ : negativer Strom

Durch einen fehlenden Gesamtstromanteil von einem Vielfachen von einem positiven Strom oder einem negativen Strom kann dann ebenso eindeutig ein Kabeldefekt detektiert werden.

Besonders bevorzugt prägt die Stromaufbringungseinheit auf jeden Nutzsignalpfad ein individuelles Signal auf.

Somit ergibt sich bei einer Anzahl von N Nutzsignalpfaden ein Gesamtsignal $I_g$, bzw. Gesamtstrom $I_g$ von:

$$I_g = I_{E1} + I_{E2} + \cdots + I_{EN}$$

Kann nun nicht der Gesamtstrom auf dem ersten Störsignalpfad gemessen werden, die Vergleichseinheit meldet aber, dass alle Elektroden verbunden sind, kann schnell und einfach aufgrund des Betrags des fehlenden Signalanteils bestimmt werden, welcher Nutzsignalpfad einen Signalpfaddefekt aufweist.

Gerade bei aufwändigen Kabelbäumen von bis zu 200 Leitungen, wie dies beispielsweise bei intrakardialen EKGs,
wie z. B. in Angiographie-Anwendungen, der Fall ist, ermöglicht dies eine gezielte Detektion der defekten Leitung. Somit kann anstatt eines kompletten hochkomplexen Kabels im Wert von bis zu 1000€ eine Einzelleitung im Wert von 10-20€ ausgetauscht werden.

Um zu überprüfen, ob die aufgeprägten Signale auf den einzelnen Nutzsignalpfaden im Messbereich liegen, umfasst die Fehlerdetektionseinheit vorzugsweise nicht eine gesamte Vergleichseinheit für alle Nutzsignalpfade, sondern weist für jeden Nutzsignalpfad eine Vergleichseinheit auf.

Bevorzugt umfassen die Vergleichseinheiten, je einen AD-Wandler, besonders bevorzugt umfassen sie aber auch je einen Komparator.

Aufgrund etwaiger weiterer Toleranzen und parasitärer Ströme im Spannungsmesssystem unterscheiden sich die aufgeprägten Signale je Nutzsignalpfad um mindestens 5 nA und/oder maximal 20 nA. Ganz besonders bevorzugt um ca. 10 nA.

Um die aufgeprägten Signale zu kontrollieren bzw. zu regeln, weist die Fehlerdetektionsvorrichtung vorzugsweise eine Stromaufbringungskontrolleinheit auf.

Um bei einem Signalpfaddefekt leicht den Nutzsignalpfad herauszufinden, welcher den defekt aufweist, ist die Stromaufbringungseinheit vorzugsweise so ausgebildet, dass sie die aufgeprägten Signale je Nutzsignalpfad einzeln schalten kann. So kann nach Detektion eines Signalpfaddefekts
z.B. schrittweise jeweils die Aufprägung eines Signals auf einen Nutzsignalpfad deaktiviert werden. Erzeugt eine Deaktivierung eines Signals auf einem Nutzsignalpfad keine Veränderung des Gesamtsignals auf dem Störsignalpfad, so weist dieser Nutzsignalpfad einen Kabeldefekt auf.

In einer besonders bevorzugten Ausgestaltung weist die Fehlerdetektionsvorrichtung einen zweiten Störsignalpfad zur Messung eines zweiten Störsignals auf. Dieser Störsignalpfad kann auf unterschiedliche Weise aufgebaut sein. Er kann derart aufgebaut sein, dass keine bioelektrischen Signale eingekoppelt werden. Es können aber vorzugsweise Störsignale eingekoppelt werden, die auch auf dem ersten Störsignalpfad auftreten, wie beispielsweise die oben beschriebenen Gleichtaktstörsignale. Der zweite Störsignalpfad kann bevorzugt zur Referenzmessung für das Störsignal auf dem ersten Störsignalpfad dienen.

Der zweite Störsignalpfad muss hierbei kein Signalmesskabel umfassen, sondern kann einer kapazitiven Messung bzw. Kopplung gegen Erde entsprechen.

Bevorzugt verläuft der zweite Störsignalpfad zwischen einem Bezugspotential des Spannungsmesssystems bzw. des EKG-Messsystems und einem externen Bezugspotential, z. B. dem Erdpotential. Diese elektrische Kopplung verläuft vorzugsweise über eine kapazitive Kopplung. Indem der zweite Störsignalpfad nur über das gemeinsame Bezugspotential mit dem Spannungsmesssystem gekoppelt ist, ist das zweite Störsignal auf dem zweiten Störsignalpfad weitgehend unabhängig von den Eingangsimpedanzen der verwendeten Kabel in den Nutzsignalpfaden. Der zweite Störsignalpfad kann daher nicht als Rückpfad für die auf Nutzsignalpfade aufgeprägten Signale dienen. Zudem wird das Störsignal aufgrund des Aufbaus des zweiten Störsignalpfads daher weitgehend von Gleichtaktstörsignalen bestimmt.

Zur Realisierung der kapazitiven Kopplung weist der zweite Störsignalpfad vorzugsweise eine mit dem Bezugspotential des Spannungsmesssystems elektrisch verbundene Leiterfläche zwischen dem Spannungsmesssystem und dem Erdpotential auf. Hierbei entspricht die Leiterfläche einer Koppelkapazität. Die Leiterfläche kann z. B. durch eine Metallplatte oder Folie

Auch der zweite Störsignalpfad kann eine Strommesseinheit aufweisen. Die Strommesseinheit kann hierbei vorzugsweise zwischen das Bezugspotential des Spannungsmesssystems und der kapazitiven Anbindung an das externe Bezugspotential der Leiterfläche geschaltet sein. Des Weiteren kann auch diese Strommesseinheit vorzugsweise einen Strommesswiderstand und eine zu diesem parallel geschaltete Spannungsmesseinrichtung aufweisen. Bei dem Strommesswiderstand handelt es sich vorzugsweise um einen Shunt-Widerstand und bei der Spannungsmesseinrichtung bevorzugt um einen Differenzverstärker.

Der zweite Störsignalpfad kann z. B. am Ausgang der Spannungsmesseinrichtung eine Störsignalerfassungseinheit aufweisen.

Sofern zwei Störsignalpfade genutzt werden, kann die Störsignalauswerteeinheit bevorzugt zwischen die beiden Störsignalerfassungseinheiten, die das erste und zweite Störsignal erfassen, gekoppelt sein. Die Störsignalauswerteeinheit kann dann vorzugsweise dazu eingerichtet sein, um ein Kombinationssignal, vorzugsweise ein Differenzsignal, aus dem ersten und zweiten Störsignal zu bilden. So kann ein Signalpfaddefekt bzw. Kabeldefekt nachgewiesen werden, wenn die Signale nicht im Differenzsignal detektiert werden können, welche auf den Nutzsignalpfaden aufgeprägt worden sind. Das Differenzsignal setzt sich also aus dem ersten und zweiten Störsignal zusammen.

Wird ein Strom als Wechselstrom auf einen Nutzsignalpfad aufgeprägt, kann es sein, dass dieser Strom dem Strom auf dem ersten Störsignalpfad sehr ähnlich ist. Wird nun ein Differenzsignal aus dem ersten und zweiten Störsignal gebildet, lassen sich die vom Nutzsignalpfad übergekoppelten Ströme leichter detektieren.

Das Kombinationssignal kann beispielsweise aber auch ein Verhältnis des ersten und zweiten Störsignals umfassen.

Vorzugsweise weist die erfindungsgemäße Fehlerdetektionsvorrichtung eine Ausgabeeinheit auf, die an den Ausgang der Störsignalauswerteeinheit angeschlossen ist und/oder extern, z. B. über eine Funkübertragung arbeitet. Die Ausgabeeinheit dient dazu, einen detektierten Signalpfaddefekt auszugeben bzw. gleich zu signalisieren. Diese Ausgabe oder Signalisierung kann dabei vor Ort z. B. optisch, akustisch erfolgen. Zudem kann die Signalisierung per Funk z. B. an einen Servicetechniker gesendet werden. Eine weitere Ausgabeform kann als Protokollierung, z. B. gemeinsam mit den Messdaten erfolgen. Besonders bevorzugt erfolgt die Protokollierung zeitlich korreliert zum Messsignal bzw. den zu messenden bioelektrischen Signalen. So kann z.B. bei einer nur zeitweise auftretenden Störung wie bei einem Wackelkontakt dokumentiert werden, welche Messwerte verwendbar sind und welche nicht.

Insbesondere wenn die Fehlerdetektionsvorrichtung in dem Spannungsmesssystem integriert ist, ist die Ausgabeeinheit bevorzugt in einer Benutzerschnittstelle des Spannungsmesssystems inkludiert. Dadurch kann beispielsweise das Bedienpersonal auf der Benutzerschnittstelle, z.B. einen Monitor, gleichzeitig die bioelektrischen Signale überprüfen und einen Kabeldefekt detektieren.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

Figur 1 schematisch eine Möglichkeit zur Positionierung der elektrischen Anschlüsse bzw. Kontakte eines EKG-Messsystems am Patienten,

Figur 2 ein Blockdiagramm für einen möglichen Ablauf des erfindungsgemäßen Verfahrens,

Figur 3 schematisch ein differentielles Spannungsmesssystem mit einer Fehlerdetektionsvorrichtung gemäß einem ersten Ausführungsbeispiel der Erfindung,

Figur 4 schematisch ein differentielles Spannungsmesssystem mit einer Fehlerdetektionsvorrichtung gemäß einem zweiten Ausführungsbeispiel der Erfindung,

Figur 5 schematisch ein differentielles Spannungsmesssystem mit einer Fehlerdetektionsvorrichtung gemäß einem dritten Ausführungsbeispiel der Erfindung,

Figur 6 grob schematisch eine Fehlerdetektionsvorrichtung nach einer der Figuren 3 bis 5 und

Figur 7 Röntgenbilder zweier EKG-Kabel mit Kinken.

In den Figuren wird exemplarisch jeweils von einem EKG-Messsystem 1 als differentielles Spannungsmesssystem 1 ausgegangen, um bioelektrische Signale BS, hier EKG-Signale BS, zu messen. Die Erfindung ist aber nicht hierauf beschränkt.

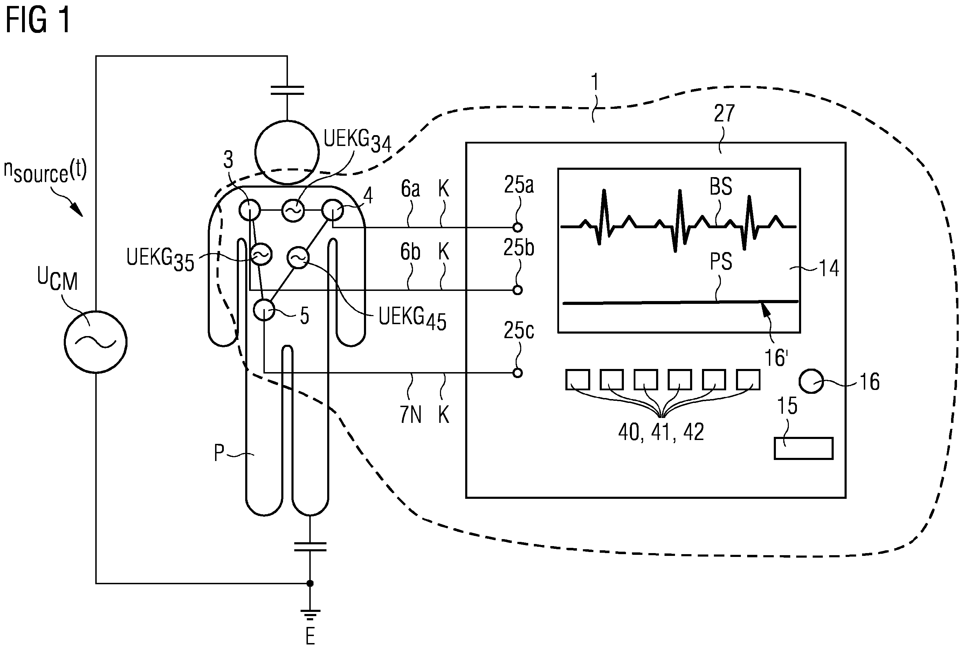

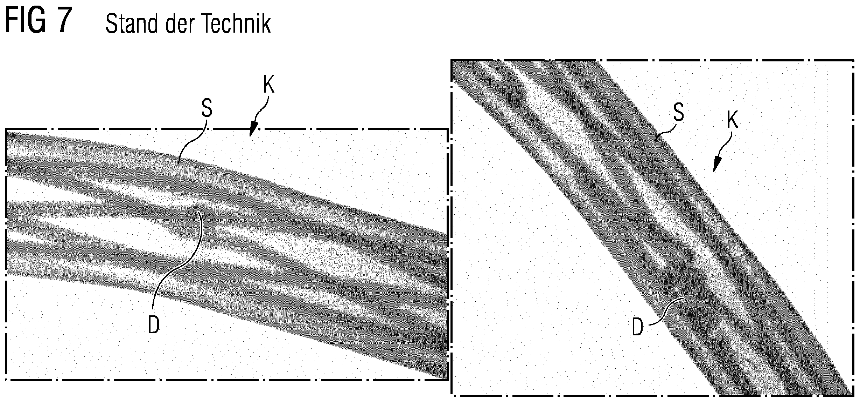

Die Figur 1 zeigt beispielhaft ein erfindungsgemäßes EKG-Messsystem 1, nämlich eine schematische Darstellung eines EKG-Geräts 27 mit seinen elektrischen Anschlüssen und daran über Kabel K angeschlossene Elektroden 3, 4, 5, um an einem Patienten P EKG-Signale BS zu messen. Dieses EKG-Messsystem 1 ist mit Hilfe der Erfindung in der Lage, einen Kabeldefekt D (wie er beispielsweise in Figur 7 zu sehen ist) in einem der Kabel K zu detektieren.

Um die EKG-Signale BS zu messen, werden mindestens eine erste Elektrode 3 und eine zweite Elektrode 4 benötigt, die auf dem Patienten P angebracht sind. Durch Signalmesskabel K sind die Elektroden 3, 4 über Anschlüsse 25a, 25b meist Steckverbindungen 25a, 25b mit dem EKG-Gerät 27 verbunden. Die erste Elektrode 3 und die zweite Elektrode 4 einschließlich der Signalmesskabel K bilden dabei einen Teil einer Signalerfassungseinheit 9 (welche später noch genauer erläutert wird) mit der die EKG-Signale BS erfasst werden können.

Eine dritte Elektrode 5 dient als Referenzelektrode, um einen Potentialausgleich zwischen dem Patienten P und dem EKG-Gerät 27 zu schaffen. Dies wird später noch genauer erläutert. Klassisch wird diese dritte Elektrode 5 am rechten Bein des Patienten angebracht (weshalb wie oben erwähnt dieser Anschluss oft auch als "Right-Leg-Drive" bzw. "RLD" bezeichnet wird). Sie kann aber, wie hier auch, an einer anderen Stelle positioniert werden. Darüber hinaus können über weitere Anschlüsse, welche in den Figuren nicht dargestellt sind, am EKG-Gerät 27 noch eine Vielzahl weiterer Kontakte für weitere Ableitungen (Potentialmessungen) am Patienten P angebracht und für die Bildung von geeigneten Signalen genutzt werden.

Zwischen den einzelnen Elektroden 3,4,5 bilden sich die Spannungspotentiale $UEKG_{34}$, $UEKG_{45}$ und $UEKG_{35}$ die zur Messung der EKG-Signale BS dienen.

Die gemessenen EKG-Signale BS werden auf einer Benutzerschnittstelle 14 des EKG-Geräts 27 angezeigt (siehe Figur 1).

Der Patient P ist bei der EKG-Messung zumindest kapazitiv mit dem Erdpotential E gekoppelt (in Figur 1 durch eine Kopplung am Kopf und dem rechten Bein schematisch dargestellt). Er unterliegt jedoch einer Störquelle $U_{cm}$, beispielsweise einem durch die Stromversorgung mit 50 Hz Wechselstrom entstehendem elektrischen Feld, und dem daraus resultierendem über den Patienten P vorliegenden und sich mit der Zeit t ständig verändernden geringen Störsignal $n_{source}(t)$, welches durch die relativ empfindliche Messung zwangsläufig mit erfasst wird. Durch diese Störquelle $U_{cm}$ werden in der Regel Störsignale über den Patienten P auf die Messleitungen in den Signalmesskabeln K eingekoppelt, auf die später noch verwiesen wird.

Die Signalmesskabel K, welche von der ersten Elektrode 3 und der zweiten Elektrode 4 zum EGK-Gerät 27 führen, sind hierbei ein Teil der Nutzsignalpfade 6a, 6b. Das Signalmesskabel K, welches von der Elektrode 5 zum EGK-Gerät 27 führt, entspricht hierbei einem Teil eines dritten Nutzsignalpfads 7N. Der dritte Nutzsignalpfad 7N überträgt Störsignale der Störquelle $U_{cm}$, welche über den Patienten P und die Elektroden eingekoppelt wurden.

Um Kabeldefekte D nun zu erkennen weist das erfindungsgemäße EKG-Messsystem 1 eine Fehlerdetektionsvorrichtung 40, welche später noch genauer erläutert wird, auf.

Mit Hilfe dieser Fehlerdetektionsvorrichtung 40 werden die Kabel K auf Kabeldefekte D überprüft.

Das durch die Fehlerdetektionsvorrichtung 40 generierte Prüfsignal PS, welches einen Kabeldefekt D signalisiert, kann wie in Figur 1 gezeigt, durch eine Ausgabeeinheit 16' auf der Benutzerschnittstelle 14 des EKG-Geräts 27 angezeigt und dargestellt werden. Hierdurch können auf der Benutzerschnittstelle 14 nicht nur die EKG- Signale BS, sondern zeitgleich auch die Kabel K auf einen eventuellen Kabeldefekt D überwacht werden.

Die Ausgabeeinheit 16 muss aber nicht zwingend in der Benutzerschnittstelle 14 integriert sein. Die Signalisierung kann z. B. auch über eine Signalleuchte, beispielsweise in Form einer LED (Leuchtdiode) oder dergleichen, realisiert werden, die einen Defekt signalisiert. Sie kann aber zusätzlich oder alternativ auch akustisch z. B. über einen Warnton erfolgen. Eine weitere Variante ist auch eine externe Übermittlung z. B. über Funk an einen Servicetechniker oder zur Ausgabe in einem Messprotokoll, um so einen Kabeldefekt D anzuzeigen bzw. zu protokollieren. Zudem kann das EKG-Gerät 27, wie in Figur 1, gezeigt eine externe Schnittstelle 15 aufweisen, um beispielsweise einen Anschluss für einen Drucker, eine Speichereinrichtung und/oder sogar ein Netzwerkbereit zu stellen.

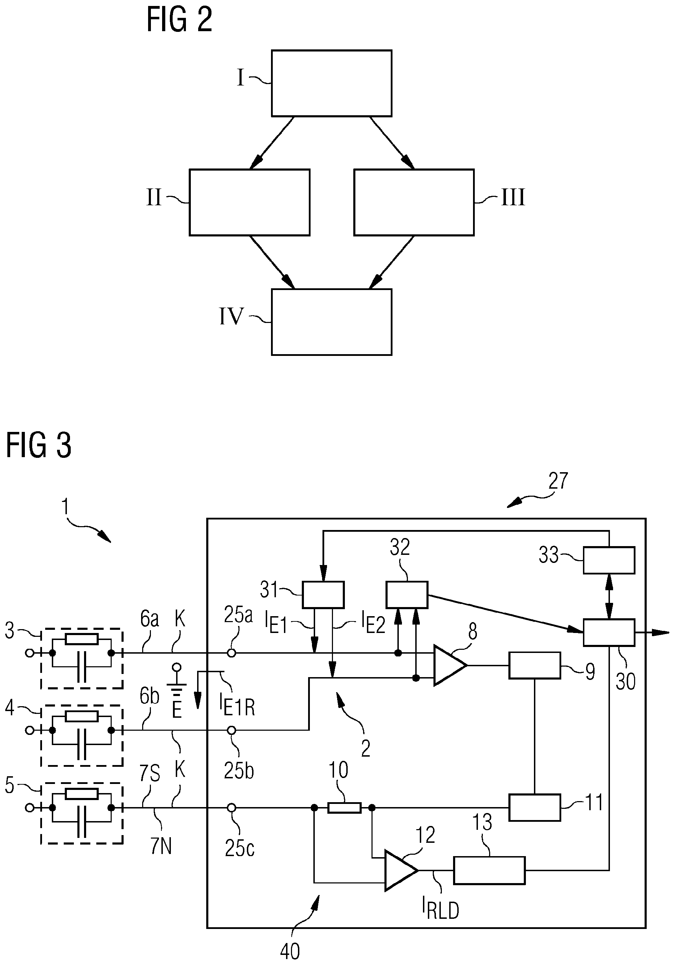

In Figur 3 ist sehr grob schematisch ein erstes Ausführungsbeispiel EKG-Geräts 27, beispielsweise des EKG-Geräts 27 aus Figur 1 eines EKG-Messsystems 1 in einem Blockschaltbild veranschaulicht.

Das EKG-Messsystem 1 umfasst eine Signalmessschaltung 2, welche zur Messung der bioelektrischen Signale BS dient.

Die Signalmessschaltung 2 hat hier, wie oben bereits erwähnt, drei Nutzsignalpfade 6a ,6b, 7N. Die Nutzsignalpfade sind, wie zu Figur 1 beschrieben, über die Elektroden 3, 4, 5, die Kabel K und die Steckverbindung 25a, 25b, 25c vom Patienten P mit dem EKG-Gerät 27 elektrisch verbunden. Die Elektroden 3, 4, 5 sind hier vereinfacht als ein RC-Glied dargestellt und veranschaulichen die Impedanz-Werte der Nutzsignalpfade 6a, 6b, 7N.

Die erste Elektrode 3 und die zweite Elektrode 4 stehen mit dem Patienten P in Kontakt. Aufgrund einer Potentialdifferenz zwischen den Ableitungsstellen, an denen die Elektroden 3, 4 am Patienten befestigt sind, wird ein Nutzsignal, z. B. ein "Herzstrom" von den Elektroden 3, 4 zu einer Verstärkerschaltung 8, z. B. einem Operationsverstärker, übertragen. Die Verstärkerschaltung 8 umfasst zwei Eingänge und ist über diese mit der ersten Elektrode 3 und zweiten Elektrode 4 elektrisch verbunden. Das Ausgangssignal der Verstärkerschaltung 8 wird an eine Signalerfassungseinheit 9 übermittelt, welche das von der Verstärkerschaltung 8 verstärkte Nutzsignal erfasst. Der erste Nutzsignalpfad 6a verläuft hierbei vom Kontakt der ersten Elektrode 3 zu dem Patienten P über die erste Elektrode 3 bis zum Eingang der Verstärkerschaltung 8. Der zweite Nutzsignalpfad 6b verläuft von dem Kontakt der zweiten Elektrode 4 zu dem Patienten P über die zweite Elektrode 4 zum Eingang der Verstärkerschaltung 8.

Die in Zusammenhang mit Figur 1 beschriebene dritte Elektrode 5 ist über das Kabel K mit einem Strommesswiderstand 10, im folgenden Shunt-Widerstand genannt, elektrisch verbunden.

Der Shunt-Widerstand 10 ist zudem mit einer Treiberschaltung 11 elektrisch verbunden, welche wie bereits erläutert auch als Right-Leg-Drive bezeichnet wird. Die Treiberschaltung 11 ist so aufgebaut, dass über die Elektrode 5 ein Referenzpotential an den Patienten angelegt wird, welches den Gleichtaktspannungen mit EKG-Anteilen entspricht. Beispielsweise kann dieses Referenzpotential in bekannter Weise auf einen inversen, verstärkten Mittelwert der Messleitungen gesetzt werden.

Dadurch kann das Referenzpotential auf die Gleichtaktspannung festgelegt werden.

Die Fehlerdetektionsvorrichtung 40 umfasst zudem eine Stromaufbringungseinheit 31. Diese kann zum einen ein erstes Signal, hier ein sich im Nanoampere-Bereich befindlicher erster Strom $I_{E1}$ auf den ersten Nutzsignalpfad 6a aufprägen. Zum anderen kann sie ein zweites Signal $I_{E2}$, hier ein sich im Nanoampere-Bereich befindlicher zweiter Strom $I_{E2}$, auf den zweiten Nutzsignalpfad 6b aufprägen. Zudem ist der zweite Strom $I_{E2}$ hier ein um 10 nA erhöhter Strom im Vergleich zum ersten Strom $I_{E1}$.

Mittels einer Stromaufbringungskontrolleinheit 33, die mit der Stromaufbringungseinheit 31 und einer Signalpfaddefektanalyseeinheit 30, die später noch beschrieben wird, kommuniziert, werden Ströme geregelt.

Bei intakten Signalmesskabeln besteht für den ersten Strom $I_{E1}$ und den zweiten Strom $I_{E2}$ außer dem Störsignalpfad 7S kein weiterer niederohmiger Rückpfad auf das gemeinsame Erdpotential.

Das heißt, dass sich auf dem Störsignalpfad 7S nicht nur die Störsignale $I_{CM}$ befinden, sondern auch der auf dem ersten Störsignalpfad 6a aufgeprägte Strom $I_{E1}$ und der auf dem zweiten Störsignalpfad 6b aufgeprägte Strom $I_{E2}$.

Es ergibt sich also bei intakten Nutzsignalpfaden 6a, 6b folgendes Störsignal $I_{RLD}$ auf dem ersten Störsignalpfad 7S:

$$I_{RLD} = I_{CM} + I_{E1} + I_{E2}$$

Die Stromaufbringungseinheit 31 wurde hier exemplarisch nur einmal dargestellt, sie kann aber beispielsweise mittels einer ersten Stromquelle, die den ersten Strom $I_{E1}$ auf den ersten Nutzsignalpfad 6a aufprägt und einer zweiten Stromquelle, die den zweiten Strom $I_{E2}$ auf den zweiten Nutzsignalpfad 6b aufprägt, realisiert werden.

Die Spannungen, welche durch die Stromaufbringungseinheit 31 an der ersten Elektrode 3 und der zweiten Elektrode 4 erzeugt wurden, befinden sich regelmäßig höchstens im Milivolt-Bereich. Denn die aufgeprägten Ströme fließen im Nanoampere-Bereich durch eine Impedanz ab, die in einem Bereich von ca.

50 kOhm bis 2 MOhm liegen kann. Diese Impedanz ist somit jedenfalls geringer als die der Nutzsignalpfade. Ist der erste Nutzsignalpfad 6a und/oder der zweite Nutzsignalpfad 6b jedoch nicht mit dem Patienten P elektrisch verbunden, so ist der Stromkreis nicht geschlossen bzw. die vom aufgeprägten Strom zu überwindende Impedanz wesentlich größer. Dadurch geht die durch die Stromquellen erzeugte Spannung an der Elektrode, die keinen Kontakt zu dem Patienten P aufweist, in Sättigung. Um dies zu überprüfen, weist die Fehlerdetektionsvorrichtung 40 eine Vergleichseinheit 32 auf. Die Vergleichseinheit 32 wurde hier zur Anschauung nur als ein Block dargestellt. Es liegt hier jedoch für den ersten Nutzsignalpfad 6a und für den zweiten Nutzsignalpfad 6b eine Vergleichseinheit vor. Die Vergleichseinheiten 32 umfassen hier Komparatoren 32. Liegen die ermittelten Ströme auf dem ersten Nutzsignalpfad 6a und auf dem zweiten Nutzsignalpfad 6b innerhalb eines vorgegebenen Messbereichs so sind die erste Elektrode 3 und die zweite Elektrode 4 mit einem Patienten elektrisch verbunden und die Komparatoren 32 melden zwei verbundene Elektroden.

Wird beispielsweise nur eine verbundene Elektrode gemeldet, so kann ein Anwender des EKG-Geräts sofort die Elektroden überprüfen und eventuell neu anbringen.

Wie zuvor beschrieben, werden die Elektroden über Signalmesskabel K an das EKG-Gerät 27 angeschlossen. Um die Anwendung des EKG-Messsystems 1 am Patienten P möglichst einfach zu gestalten, sollen die Kabel K schlank, leicht und zugleich geschirmt sein. Diese Merkmalskombination führen jedoch oft zu Kabeldefekten D wie sie auf den Röntgenbildern zweier Kabel K, die z. B. in einem EKG-Gerät 27 zur Messung eines EKG-Signals BS Anwendung finden, in Figur 7 zu sehen sind. Die beiden Kabel K zeigen hierbei jeweils einen Kabeldefekt D in Form einer Kinke D. Dieser Kabeldefekt D kann nach einer Biegung oder Torsion der Kabel K entstehen. Hierbei kann es zu einer irreversiblen Auswölbung der Messleitungen kommen, welche die Leitungs-Isolation durchbricht. Durch die Durchbrechung der Leitungs-Isolation kann ein Kontakt zwischen den Messleitungen und der Schirmung S entstehen. Durch diesen Kontakt kommt es zu einer Reduzierung der Eingangsimpedanz und zu einer Verstärkung von Störungen.

Durch die Reduzierung der Eingangsimpedanz des Kabels K bei einem Kabeldefekt D, kommt es zu einem weiteren niederohmigen Rückflusspfad für den jeweiligen aufgeprägten Strom. Liegt beispielsweise ein Kabeldefekt D des ersten Nutzsignalpfads 6a vor, so fließt der Strom $I_{E1}$ über einen Rückpfad $I_{E1R}$ in Erde E (was hier exemplarisch eingezeichnet wurde) und erhöht somit nicht mehr den Strom $I_{RLD}$ auf dem Störsignalpfad 7S. Der Komparator 32 erkennt diesen fehlerhaft abgeflossenen Strom aber nicht und gibt weiterhin an, dass die Elektrode 3 mit dem Patienten P elektrisch verbunden ist.

Um nun diesen Kabeldefekt D detektieren zu können, weist das erfindungsgemäße Spannungsmesssystem 1 zur oben beschriebenen Signalmessschaltung 2, ein erstes Ausführungsbeispiel einer erfindungsgemäßen Fehlerdetektionsvorrichtung 40 auf, welche den Störsignalpfad 7S umfasst.

Die Nutzsignalpfade 6a, 6b umfassen die erste Elektrode 3 und die zweite Elektrode 4, die Kabel K, die Stromaufbringungseinheit 31, die Vergleichseinheit 32 sowie die weitere geräteinterne Leitung (mit der Verstärkerschaltung 8) bis zur Signalerfassungseinheit 9 hat hier ebenfalls eine Doppelfunktion. Sie gehören nämlich zum einen zur Signalmessschaltung 2, um bioelektrische Signale BS zu messen. Zum anderen gehören sie zur Fehlerdetektionsvorrichtung 40, 41, 42 um zu überprüfen, ob oder gegeben falls wie viele Elektroden der entsprechenden Nutzsignalpfade 6a, 6b mit einem Patienten verbunden bzw. defekt sind.

Das von der Störsignalauswerteeinheit 13 ausgegebene Signal $I_{RLD}$ wird in einer Signalpfaddefektanalyseeinheit 30 zusammen mit den Daten der Vergleichseinheit 32 analysiert. Melden die Vergleichseinheiten 32, dass alle Elektroden verbunden sind und die Störsignalauswerteeinheit 13 gibt einen Strom $I_{RLD}$ aus, welcher die Störsignale $I_{CM}$ sowie den ersten aufgeprägten Strom $I_{E1}$ und den zweiten aufgeprägten Strom $I_{E2}$ umfasst, so detektiert die Signalpfaddefektanalyseeinheit 30, dass alle Elektroden mit dem Patienten P verbunden sind und kein Kabeldefekt D vorliegt.

Melden die Vergleichseinheiten 32, dass alle Elektroden 3, 4 verbunden sind, aber die Störsignalauswerteeinheit 13 gibt einen Strom $I_{RLD}$ aus, welcher die Störsignale $I_{CM}$ sowie den ersten aufgeprägten Strom $I_{E1}$ aber nicht den zweiten aufgeprägten Strom $I_{E2}$ umfasst, so detektiert die Signalpfaddefektanalyseeinheit 30, dass alle Elektroden 3, 4 mit dem Patienten P verbunden sind, dass aber ein Kabeldefekt D vorliegt. Analog funktioniert dies natürlich auch, wenn beide Nutzsignalpfade 6a, 6b einen Signalpfaddefekt D aufweisen oder wenn nur der erste Nutzsignalpfad 6a einen Kabeldefekt D aufweist. Wenn sich wie hier der erste Strom $I_{E1}$ und der zweite Strom $I_{E2}$ unterscheiden, kann die Signalpfaddefektanalyseeinheit 30 gezielt angeben, welches Signalpfadkabel K einen Defekt D aufweist.

Die Fehlerdetektionsvorrichtung 40 muss aber nicht, wie z. B. in Figur 3 gezeigt, in dem EKG-Messsystem integriert sein. Sie kann auch in ein bereits bestehendes EKG-Messsystem über beispielsweise Steckverbindungen, eingebaut werden oder auch vor- oder zwischengeschaltet sein. Durch eine solche Nachrüstung ist es möglich, auch mit einem bereits bestehendem EKG-Messsystem Kabeldefekte D zu detektieren.

Um die EKG-Signale BS sowie eventuelle Kabeldefekte D mittels eines Prüfsignals PS (siehe Figur 1) auf der Benutzerschnittstelle 14 parallel anzuzeigen, ist diese an die Signalerfassungseinheit 9 der Signalmessschaltung 2 und an die Signalpfaddefektanalyseeinheit 30 der Fehlerdetektionsvorrichtung 40 angeschlossen.

Dies ist in Figur 1 grob schematisch veranschaulicht. Die Benutzerschnittstelle 14 ist daher in Figur 1 mit einer Ausgabeeinheit 16' gezeigt, um diese Möglichkeit darzustellen.

Die oben beschriebene weitere Ausgabeeinheit 16 zur beispielsweise optischen und/oder akustischen Signalisierung eines Kabeldefekts D kann ebenfalls mit einem Ausgang der Störsignalauswerteeinheit 13 und der Signalpfaddefektanalyseeinheit 30 gekoppelt werden.

Zudem ist das differentielle Spannungsmesssystem 1 wie schon erwähnt mit einer externen Schnittstelle 15 beispielsweise für ein Netzwerk, einen Drucker und/oder einen Speichert etc. ausgestattet, die z. B. mit der Signalerfassungseinheit 9 der Signalmessschaltung 2 und/oder Signalpfaddefektanalyseeinheit 30 signaltechnisch verbunden sein kann.

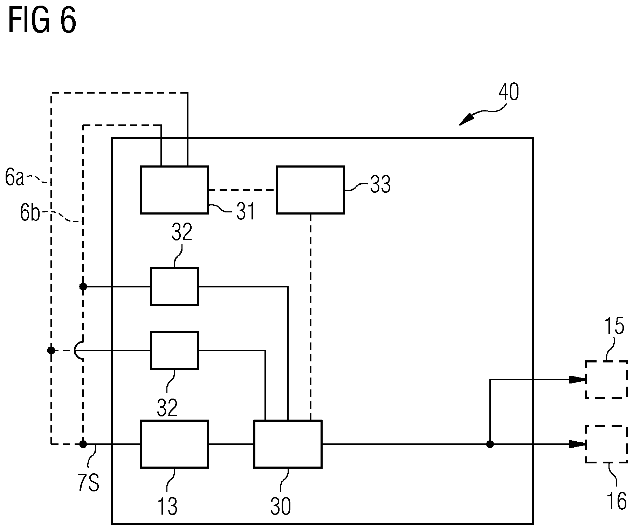

Figur 6 zeigt hierzu wie die Signalpfaddefektanalyseeinheit 30 elektrisch mit der externen Schnittstelle 15 und der Ausgabeeinheit 16 verbunden sein kann.

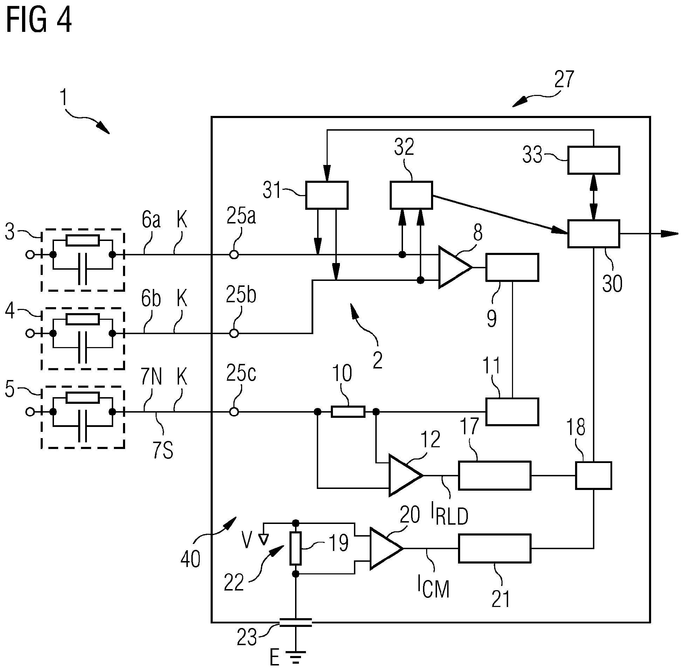

In Figur 4 wird eine weitere erfindungsgemäße Ausgestaltung des Spannungsmesssystems 1 bzw. des EKG-Messsystems 1 ähnlich dem in Figur 3, gezeigt. Dieses differentielle Spannungsmesssystem 1 umfasst ebenfalls eine Signalmessschaltung 2 und eine Fehlerdetektionsvorrichtung 41.

Die Signalmessschaltung 2 entspricht der in Zusammenhang mit Figur 2 beschriebenen Signalmessschaltung 2.

Die Fehlerdetektionsvorrichtung 41 stellt ein weiteres Ausführungsbeispiel der Erfindung dar, um die Signalpfaddefekte D, bzw. Kabeldefekte D, zu detektieren. Die Fehlerdetektionsvorrichtung 41 weist hierbei zwei Störsignalpfade 7S, 22 auf. Der erste Störsignalpfad 7S umfasst, wie bei dem Ausführungsbeispiel in Figur 2, die dritte Elektrode 5, welche mit ihrem Eingang an einen Patienten P angeschlossen ist und verläuft bis zu dem Shunt-Widerstand 10, der mit dem Ausgang der Elektrode 5 elektrisch verbunden ist. Die über dem Shunt-Widerstand 10 abfallende Spannung wird auch hier von der parallel zum Shunt-Widerstand 10 geschalteten ersten Spannungsmesseinrichtung 12 gemessen. Das dadurch gemessene Störsignal $I_{RLD}$ wird anschließend von einer an den Ausgang der ersten Spannungsmesseinrichtung 12 geschalteten ersten Störsignalerfassungseinheit 17 digitalisiert, weiter verarbeitet und erfasst.

Die Fehlerdetektionsvorrichtung 41 umfasst hier zusätzlich eine zweite Strommesseinheit 19, 20. Mit dieser zweiten Strommesseinheit 19, 20 wird der von einem internen Bezugspotential V des EKG-Geräts 27 über eine kapazitive Kopplung zu einem externen festen Bezugspotential E, dem Erdpotential E, fließende Strom gemessen. Bei diesem zweiten gemessenen Störsignal $I_{CM}$ handelt es sich vor allem wieder um Gleichtaktstörsignale. Die kapazitive Kopplung zwischen dem EKG-Gerät 27 und dem Erdpotential E ist ohnehin immer vorhanden. Um einen definierten Störsignalpfad 22 für dieses Störsignal $I_{CM}$ zur Verfügung zu stellen, an dem das Störsignal $I_{CM}$ gut gemessen werden kann, wird eine großflächigere Leiterfläche 23, z. B. in Form einer Metallplatte oder einer Folie, mit dem internen Bezugspotential V des EKG-Geräts 27 verbunden, welche eine "Kondensatorfläche" gegen das Erdpotential E bildet. Die zweite Strommesseinheit 19, 20 ist in diesem zweiten Störsignalpfad 22 zwischen dem internem Bezugspotential V und der Leiterfläche 23 geschaltet.

Für die zweite Strommesseinheit 19, 20, wird zur Strommessung auf dem zweiten Störsignalpfad 22 ein zwischen internes Bezugspotential V und Leiterfläche 23 geschalteter Strommesswiderstand 19, im weiteren zweiter Shunt-Widerstand genannt, verwendet und eine zu diesem parallel geschaltete zweite Spannungsmesseinrichtung 20. Die zweite Spannungsmesseinrichtung 20 kann dabei wieder durch einen Verstärker, z. B. durch einen PGA, realisiert werden.

Das gemessene zweite Störsignal $I_{CM}$ wird durch eine an den Ausgang der Spannungsmesseinrichtung 20 geschaltete Störsignalerfassungseinheit 21 erfasst, z. B. durch einen A/D-Wandler digitalisiert und gegebenenfalls weiter verarbeitet.

Das erste Störsignal $I_{RLD}$, welches Gleichtaktstörsignale und bei intakten Signalkabeln K des ersten Nutzsignalpfads 6a und des zweiten Nutzsignalpfads 6b, die übergekoppelten Ströme $I_{E1}$, $I_{E2}$ enthält, wird mit dem zweiten Störsignal $I_{CM}$, welches nur Gleichtaktstörsignale enthält, in einer - hier vorzugsweise digital arbeitenden - Störsignalauswerteeinheit 18 gemeinsam ausgewertet. Die Störsignalauswerteeinheit 18 ist hierzu mit den beiden Störsignalerfassungseinheiten 17, 21 elektrisch verbunden.

Die Störsignalauswerteeinheit 18 ist hierbei eingerichtet, um das erste Störsignal $I_{RLD}$ und das zweite Störsignal $I_{CM}$ zu verarbeiten. Dadurch können die Gleichtaktstörungen auf dem ersten Störsignalpfad 7S von den dort bei intakten Kabeln K bzw. Messleitungen K auftretenden übergekoppelten Strömen $I_{E1}$, $I_{E2}$ getrennt bzw. unterschieden werden. Infolge dessen kann ein Kabeldefekt D leichter nachgewiesen werden. Zur Auswertung der Störsignale $I_{RLD}$, $I_{CM}$ die ja hier digital vorliegen, kann die Störsignalauswerteeinheit 18 wieder durch eine Recheneinrichtung mit geeigneter Software realisiert werden und/oder beispielsweise auch durch einen oder mehrere ASIC.

Vorzugsweise kann die Störsignalauswerteeinheit 18 so ausgebildet sein, dass aus den beiden Störsignalen $I_{RLD}$, $I_{CM}$ ein Ausgangssignal erzeugt wird, in dem die Gleichtaktstörungen auf dem ersten Störsignalpfad 7S eliminiert sind. Dadurch verbleiben nur die bei intakten Kabeln auftretenden Ströme $I_{E1}$, $I_{E2}$, die auf den ersten Nutzsignalpfad 6a und den zweiten Nutzsignalpfad 6b aufgeprägt worden sind.

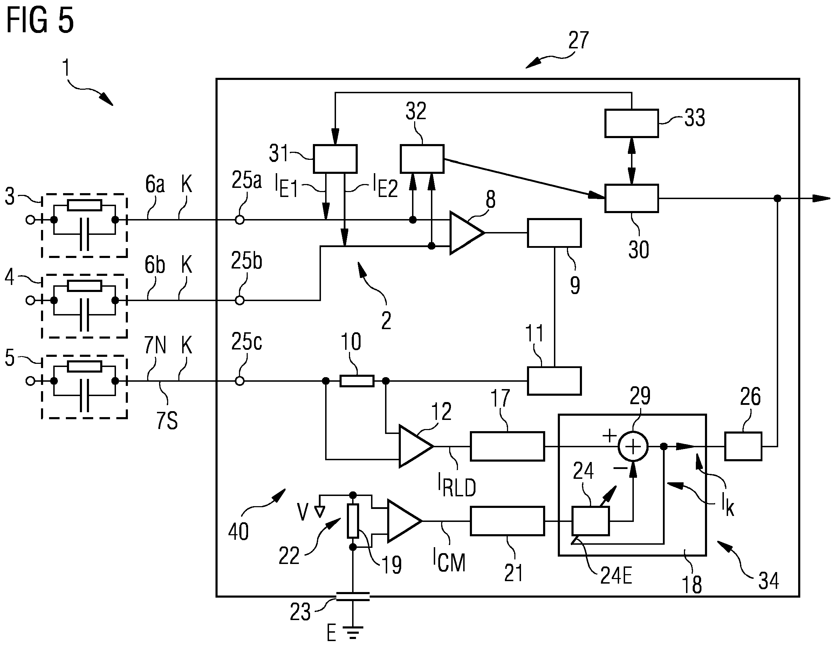

Ein Teil dieser Störsignalauswerteeinheit 18 kann hierzu bei einer besonders bevorzugten Variante der Fehlerdetektionsvorrichtung 42, wie sie in Figur 5 gezeigt ist, mit Hilfe einer Filtereinrichtung 34 realisiert werden. Diese Filtereinrichtung 34 filtert das zweite Störsignal $I_{CM}$ so, dass bei einer Differenzbildung des ersten und zweiten Störsignals $I_{RLD}$, $I_{CM}$ das resultierende Differenzsignal bei einem intaktem Signalmesskabel K nur die durch die Stromaufbringungseinheit 31 induzierten Ströme aufweist (gegebenenfalls mit üblichem Messrauschen).

In der Filtereinrichtung 34 durchläuft das zweite Störsignal $I_{CM}$ einer Filterung durch einen adaptiven Filter 24. Ein anschließendes Summierglied 29 führt eine Differenzbildung des ersten Störsignals $I_{RLD}$ (welches unverändert bleibt) und zweiten Störsignals $I_{CM}$ durch. Der verwendete adaptive Filter 24 wird hierbei abhängig von den beiden Störsignale $I_{RLD}$, $I_{CM}$ bzw. deren Differenz eingestellt. Hierzu wird bevorzugt ein Stellwerteingang 24E, welcher mit dem Ausgang des Summierglieds 29 verbunden ist, mit dem entstehendem Differenzsignal $I_k$ (als ein Kombinationssignal $I_k$) der beiden Störsignale $I_{RLD}$, $I_{CM}$ beaufschlagt.

Optional kann das dadurch entstehende Differenzsignal $I_k$ durch eine an den Ausgang der Filtereinrichtung 30 gekoppelte Signalverarbeitungseinheit 26 weiterverarbeitet, z. B. geglättet, werden und bildet dann das Prüfsignal PS. Bei keinem Kabeldefekt D enthält das Prüfsignal PS dann nur noch die durch die Nutzsignalpfade 6a, 6b eingekoppelten Ströme $I_{E1}$, $I_{E2}$. Die Signalverarbeitungseinheit 26 kann dann beispielweise nach diesen Strömen $I_{E1}$, $I_{E2}$ suchen, um zu bestimmen, ob die Messleitungen K intakt sind. Diese Auswertung kann beispielsweise über eine Schwellwert-Analyse erfolgen. Es können aber auch eine Vielzahl von weiteren Algorithmen verwendet werden, wie beispielsweise eine Mustererkennung oder ein Kalman-Filter, um das Differenzsignal $I_k$ bzw. Kombinationssignal $I_k$ auszuwerten. Auch eine Analyse im Zeit- oder Frequenzbereich ist möglich.

Die Signalmessschaltung 2 in Figur 5 und der weitere Aufbau der Fehlerdetektionsvorrichtung 42 entsprechen dem Aufbau aus Figur 3. Zur besseren Übersicht wurden hier aber die Benutzerschnittstelle 14, die externe Schnittstelle 15 und die Ausgebeeinheit 16 nicht dargestellt.

Die erfindungsgemäßen Fehlerdetektionsvorrichtungen 40, 41, 42 erlauben es also, einen Kabeldefekt D in einem EKG-System 1 sofort und eindeutig detektieren zu können. Hierfür muss kein gesondertes Testverfahren von einem geschulten Servicetechniker durchgeführt werden. Die Überprüfung der Kabel K verläuft simultan zur EKG-Messung und Defekte D können von jedem Bedienpersonal des EKG-Geräts schnell und einfach detektiert werden. Werden zudem unterschiedliche Ströme auf die Nutzsignalpfade aufgeprägt, ist es auch möglich, zu bestimmen, welcher Nutzsignalpfad einen Signalpfaddefekt aufweist.

In Figur 2 ist ein Blockdiagramm gezeigt, mit dem ein Verfahren gemäß einem Ausführungsbeispiel der Erfindung veranschaulicht wird.

In einem ersten Verfahrensschritt I werden also Ströme auf die Nutzsignalpfade eines Spannungsmesssystems aufgeprägt.

In einem darauf folgenden Verfahrenschritt II wir überprüft, ob die Ströme auf den Nutzsignalpfaden in einem Messbereich liegen. Liegen die Ströme nicht im Messbereich, d.h. sie können nicht erfasst werden, so liegt mindestens eine Elektrode eines Nutzsignalpfads nicht an einem Patienten an.

Daraufhin kann das Verfahren gestoppt werden und ein Anwender kann den Sitz der Elektroden überprüfen und diese neu anbringen. Danach kann der Verfahrensschritt II erneut wiederholt werden.

Liegen die Ströme dann im Messbereich, so weisen die Elektroden einen Kontakt zu einem Patienten auf. Daraufhin erfolgt in Verfahrensschritt III eine Strommessung auf dem ersten Störsignalpfad 7S. Sind die Elektroden der Nutzsignalpfade an einen Patienten P angeschlossen, wie dies im Verfahrensschritt 2 überprüft worden ist, so fließen die aufgeprägten Ströme ebenfalls über den Störsignalpfad 7S und können gemessen werden.

Der Verfahrensschritt II und der Verfahrensschritt III müssen nicht hintereinander verlaufen. Sie können auch zeitgleich nebeneinander stattfinden.

In Verfahrensschritt IV werden die auf dem Störsignal 7S detektierten Ströme $I_{RLD}$ mit den Ergebnissen aus Verfahrensschritt II verglichen.

Liegen die überprüften Ströme, die auf die Nutzsignalpfade 6a, 6b aufgeprägt worden sind, im Messbereich und es wurden beispielsweise zwei verbundene Elektroden detektiert und die Strommessung auf dem ersten Störsignalpfad misst ebenfalls die Ströme die über zwei Elektroden auf den Störsignalpfad eingekoppelt wurden, so liegen beiden Elektroden an und es liegt kein Signalpfaddefekt vor.

Liegen die überprüften Ströme, die auf die Nutzsignalpfade 6a, 6b aufgeprägt worden sind, im Messbereich und es wurden beispielsweise zwei verbundene Elektroden detektiert, aber die Strommessung auf dem ersten Störsignalpfad misst beispielsweise nur einen Strom der über eine Elektrode auf den Störsignalpfad eingekoppelt wurde, so liegt ein Signalpfaddefekt eines Nutzsignalpfads vor.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können. So kann es sich bei dem differentiellen Spannungsmesssystem nicht nur um ein EKG-Gerät handeln, sondern auch um andere medizinische Geräte mit denen bioelektrische Signale erfasst werden, wie beispielsweise EEGs , EMGs usw. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können. Die Erfindung wird in den anhängenden Ansprüchen definiert.

**Patentansprüche**

1. Fehlerdetektionsvorrichtung (40; 41; 42) zur Detektion von Signalpfaddefekten in einem Spannungsmesssystem (1) mit einer Signalmessschaltung (2) zur Messung von bioelektrischen Signalen (BS) mit einer Anzahl von Nutzsignalpfaden (6a; 6b), wobei die Fehlerdetektionsvorrichtung (40; 41; 42) zumindest folgende Komponenten aufweist:

   - zumindest eine Stromaufbringungseinheit (31), wobei die Stromaufbringungseinheit (31) so ausgebildet ist, dass sie ein Signal ($I_{E1}$; $I_{E2}$) auf einen ersten Nutzsignalpfad (6a; 6b) aufprägt, wobei der aufgeprägte Strom im Nanoampere-Bereich liegt, um gemessene bioelektrische Signale nicht zu verfälschen,
   - zumindest eine erste Vergleichseinheit (32), welche überprüft, ob das Signal des ersten Nutzsignalpfads (6a; 6b) innerhalb eines Messbereichs liegt, bei dem davon ausgegangen wird, dass das Signal auf dem Nutzsignalpfad fließt,
   - zumindest einen als Strommesspfad ausgebildeten ersten Störsignalpfad (7S) zur stromerfassenden Messung eines ersten Störsignals ($I_{RLD}$)
   - und eine Signalpfaddefektanalyseeinheit (30), welche ausgebildet ist, um einen Signalpfaddefekt (D) in einem Nutzsignalpfad (6a; 6b) des Spannungsmesssystems (1) zu detektieren, wenn das aufgeprägte Signal ($I_{E1}$; $I_{E2}$) nicht auf dem ersten Störsignalpfad (7S) gemessen wird und das überprüfte Signal der Vergleichseinheit (32) innerhalb des Messbereichs liegt.

2. Fehlerdetektionsvorrichtung nach Anspruch 1, wobei die Stromaufbringungseinheit (31) so ausgebildet ist, das sie auf die Nutzsignalpfade (6a; 6b) unterschiedliche Signale ($I_{E1}$; $I_{E2}$) aufprägt.

3. Fehlerdetektionsvorrichtung nach einem der vorstehenden Ansprüche, umfassend eine Stromaufbringungskontrolleinheit (33), welche die aufgeprägten Signale ($I_{E1}$; $I_{E2}$) auf die Nutzsignalpfade (6a; 6b) der Stromaufbringungseinheit (30) kontrolliert.

4. Fehlerdetektionsvorrichtung nach einem der vorstehenden Ansprüche, wobei die Stromaufbringungseinheit (31) so ausgebildet ist, dass sie einen Wechselstrom und/oder einen Gleichstrom auf die Nutzsignalpfade (6a; 6b) aufprägt.

5. Fehlerdetektionsvorrichtung nach einem der vorstehenden Ansprüche, wobei die Stromaufbringungseinheit (31) so ausgebildet ist, dass sie die aufgeprägten Ströme ($I_{E1}$; $I_{E2}$) je Nutzsignalpfad (6a; 6b) einzeln schaltet.

6. Fehlerdetektionsvorrichtung nach einem der vorstehenden Ansprüche, wobei die Stromaufbringungseinheit (31) so ausgebildet ist, dass die aufgeprägten Signale ($I_{E1}$; $I_{E2}$) auf die Nutzsignalpfade (6a; 6b) positive Ströme ($I_{E1}$; $I_{E2}$) umfassen.

7. Fehlerdetektionsvorrichtung nach einem der vorstehenden Ansprüche, wobei die Stromaufbringungseinheit (31) so ausgebildet ist, dass auf eine Anzahl von Nutzsignalpfaden ein positiver Strom und auf eine Anzahl von Nutzsignalpfaden ein negativer Strom aufgeprägt wird.

8. Fehlerdetektionsvorrichtung nach einem der vorstehenden Ansprüche, wobei die aufgeprägten Signale ($I_{E1}$; $I_{E2}$) je Nutzsignalpfad (6a; 6b) sich um mindestens 5 nA und/oder maximal 20 nA unterscheiden.

9. Fehlerdetektionsvorrichtung nach einem der vorstehenden Ansprüche, aufweisend einen zweiten Störsignalpfad (22) zur Messung eines zweiten Störsignals ($I_{CM}$).

10. Fehlerdetektionsvorrichtung nach Anspruch 9, mit einer Störsignalauswerteeinheit (18), wobei die Störsignalauswerteeinheit (18) so ausgebildet ist, um ein Kombinationssignal ($I_k$), vorzugsweise ein Differenzsignal ($I_k$), des ersten Störsignals ($I_{RLD}$) und des zweiten Störsignals ($I_{CM}$) zu bilden.

11. Spannungsmesssystem (1), mit mindestens einer Signalmessschaltung (2) mit einer Anzahl von Nutzsignalpfaden (6a; 6b) zur Messung von bioelektrischen Signalen (BS) und mit einer Fehlerdetektionsvorrichtung (40; 41; 42) nach einem der vorstehenden Ansprüche.

12. Spannungsmesssystem nach Anspruch 11, aufweisend zumindest zwei Nutzsignalpfade (6a; 6b).

13. Verfahren zur Detektion von Signalpfaddefekten (D) in einem Spannungsmesssystem (1) zur Messung von bioelektrischen Signalen (BS), wobei die bioelektrischen Signale (BS) mittels einer Signalmessschaltung (2) mit einer Anzahl von Nutzsignalpfaden (6a; 6b) gemessen werden, wobei das Verfahren zumindest die folgenden Schritte umfasst:

- Aufprägen zumindest eines ersten Signals ($I_{E1}$; $I_{E2}$) auf zumindest einen ersten Nutzsignalpfad (6a; 6b) mittels zumindest einer ersten Stromaufbringungseinheit (31), derart, dass der aufgeprägte Strom im Nanoampere-Bereich liegt, um gemessene bioelektrische Signale nicht zu verfälschen,
- Überprüfung mittels zumindest einer ersten Vergleichseinheit (32), ob das Signal ($I_{E1}$; $I_{E2}$) des ersten Nutzsignalpfads (6a; 6b) innerhalb eines Messbereichs liegt, bei dem davon ausgegangen wird, dass das Signal auf dem Nutzsignalpfad fließt,
- stromerfassende Messung zumindest eines ersten Störsignals ($I_{RLD}$) auf zumindest einem als Strommesspfad ausgebildeten ersten Störsignalpfad (7S),
- Analyse mittels einer Signalpfaddefektanalyseeinheit (30), um einen Signalpfaddefekt (D) in einem Nutzsignalpfad (6a; 6b) des Spannungsmesssystems (1) zu detektieren, wenn das aufgeprägte Signal ($I_{E1}$; $I_{E2}$) nicht auf dem ersten Störsignalpfad (7S) gemessen wird und das überprüfte Signal ($I_{E1}$'; $I_{E2}$') der Vergleichseinheit (32) innerhalb des Messbereichs liegt.

14. Computerprogrammprodukt mit einem Computerprogramm, welches direkt in eine Speichereinrichtung eines Spannungsmesssystems (1) nach Anspruch 11 ladbar ist, mit Programmabschnitten, um alle Schritte des Verfahrens nach Anspruch 13 auszuführen, wenn das Computerprogramm in dem Spannungsmesssystem (1) ausgeführt wird.

15. Computerlesbares Medium, auf welchem ein Computerprogramm nach Anspruch 14 gespeichert ist.

**Claims**

1. Fault detection device (40; 41; 42) for the detection of signal path defects in a voltage measuring system (1) having a signal measuring circuit (2) for measuring bioelectric signals (BS) with a number of useful signal paths (6a; 6b),
   wherein the fault detection device (40; 41; 42) has at least the following components:

   - at least one electricity generating unit (31), wherein the electricity generating unit (31) is designed such that it impresses a signal ($I_{E1}$; $I_{E2}$) on a first useful signal path (6a; 6b), wherein the impressed current is in the nanoampere range, in order not to falsify measured bioelectric signals,
   - at least one first comparison unit (32), which checks whether the signal of the first useful signal path (6a; 6b) lies within a measuring range, in which it is assumed that the signal flows on the useful signal path,
   - at least one first interference signal path (7S), designed as a current measurement path, for current-detecting measurement of a first interference signal ($I_{RLD}$)
   - and a signal path defect analysis unit (30), which is designed to detect a signal path defect (D) in a useful signal path (6a; 6b) of the voltage measuring system (1) when the impressed signal ($I_{E1}$; $I_{E2}$) is not measured on the first interference signal path (7S) and the checked signal of the comparison unit (32) lies within the measuring range.

2. Fault detection device according to claim 1, wherein the electricity generating unit (31) is designed such that it impresses different signals ($I_{E1}$; $I_{E2}$) on the useful signal paths (6a; 6b).

3. Fault detection device according to one of the preceding claims, comprising an electricity generating checking unit (33), which checks the impressed signals ($I_{E1}$; $I_{E2}$) on the useful signal paths (6a; 6b) of the electricity generating unit (30).

4. Fault detection device according to one of the preceding claims, wherein the electricity generating unit (31) is designed such that it impresses an alternating current and/or a direct current on the useful signal paths (6a; 6b).

5. Fault detection device according to one of the preceding claims, wherein the electricity generating unit (31) is designed such that it switches the impressed currents ($I_{E1}$; $I_{E2}$) individually per useful signal path (6a; 6b).

6. Fault detection device according to one of the preceding claims, wherein the electricity generating unit (31) is designed such that the impressed signals ($I_{E1}$; $I_{E2}$) on the useful signal paths (6a; 6b) comprise positive currents ($I_{E1}$; $I_{E2}$).

7. Fault detection device according to one of the preceding claims, wherein the electricity generating unit (31) is designed such that a positive current is impressed on a number of useful signal paths and a negative current is impressed on a number of useful signal paths.

8. Fault detection device according to one of the preceding claims, wherein the impressed signals ($I_{E1}$; $I_{E2}$) differ by at least 5 nA and/or a maximum of 20 nA per useful signal path (6a; 6b).

9. Fault detection device according to one of the preceding claims, having a second interference signal path (22) for measuring a second interference signal ($I_{CM}$).

10. Fault detection device according to claim 9, having an interference signal evaluation unit (18), wherein the interference signal evaluation unit (18) is designed to form a combination signal ($I_k$), preferably a differential signal ($I_k$), of the first interference signal ($I_{RLD}$) and the second interference signal ($I_{CM}$).

11. Voltage measuring system (1), having at least one signal measuring circuit (2) with a number of useful signal paths (6a; 6b) for measuring bioelectric signals (BS) and having a fault detection device (40; 41; 42) according to one of the preceding claims.

12. Voltage measuring system according to claim 11, having at least two useful signal paths (6a; 6b).

13. Method for the detection of signal path defects (D) in a voltage measuring system (1) for measuring bioelectric signals (BS), wherein the bioelectric signals (BS) are measured by means of a signal measuring circuit (2) having a number of useful signal paths (6a; 6b), wherein the method comprises at least the following steps:

- impressing at least one first signal ($I_{E1}$; $I_{E2}$) on at least one first useful signal path (6a; 6b) by means of at least one first electricity generating unit (31), in such a manner that the impressed current is in the nanoampere range, in order not to falsify measured bioelectric signals,
- checking by means of at least one first comparison unit (32), whether the signal ($I_{E1}$; $I_{E2}$) of the first useful signal path (6a; 6b) lies within a measuring range, in which it is assumed that the signal flows on the useful signal path
- current-detecting measurement of at least one first interference signal ($I_{RLD}$) on at least one first interference signal path (7S) designed as a current measuring path
- analysis by means of a signal path defect analysis unit (30) in order to detect a signal path defect (D) in a useful signal path (6a; 6b) of the voltage measuring system (1) when the impressed signal ($I_{E1}$; $I_{E2}$) is not measured on the first interference signal path (7S) and the checked signal ($I_{E1}'$; $I_{E2}'$) of the comparison unit (32) lies within the measuring range.

14. Computer program product having a computer program, which can be loaded directly into a storage device of a voltage measuring system (1) according to claim 11, having program segments in order to carry out all steps of the method according to claim 13 when the computer program is carried out in the voltage measuring system (1).

15. Computer-readable medium, on which a computer program according to claim 14 is stored.

**Revendications**

1. Dispositif (40 ; 41 ; 42) de détection de défaut pour la détection de défaillances de voie de signal dans un système (1) de mesure de tension comprenant un circuit (2) de mesure du signal pour la mesure de signaux (BS) bioélectriques ayant un certain nombre de voies (6a ; 6b) de signal utile, dans lequel le dispositif (40 ; 41 ; 42) de détection de défaut comporte au moins les composants suivants :

- au moins une unité (31) d'application du courant, dans lequel l'unité (31) d'application du courant est constituée de manière à appliquer un signal ($I_{E1}$ ; $I_{E2}$) à une première voie (6a ; 6b) de signal utile, dans lequel le courant imprimé est dans le domaine du nanoampère afin de ne pas fausser la mesure de signaux bioélectriques,
- au moins une première unité (32) de comparaison, qui contrôle si le signal de la première voie (6a ; 6b) de signal utile se trouve dans une plage de mesure, dans laquelle on part du fait que le signal passe sur la voie de signal utile,
- au moins une première voie (7S) de signal parasite constituée en voie de mesure du courant pour la mesure par détection de courant d'un premier signal ($I_{RLD}$) parasite,
- et une unité (30) d'analyse de défaillance de voie de signal, qui est constituée pour détecter une défaillance (D) de voie de signal dans une voie (6a ; 6b) de signal utile du système (1) de mesure de tension, si le signal ($I_{E1}$ ; $I_{E2}$) imprimé n'est pas mesuré sur la première voie (7S) de signal parasite et si le signal contrôlé de l'unité (32) de comparaison se trouve dans la plage de mesure.

2. Dispositif de détection de défaut suivant la revendication 1, dans lequel l'unité (31) d'application du courant est constituée de manière à imprimer des signaux ($I_{E1}$ ; $I_{E2}$) différents sur les voies (6a ; 6b) de signal utile.

3. Dispositif de détection de défaut suivant l'une des revendications précédentes, comprenant une unité (33) de commande de l'application du courant, qui commande les signaux ($I_{E1}$ ; $I_{E2}$) imprimés sur les voies (6a ; 6b) de signal utile de l'unité (30) d'application du courant.

4. Dispositif de détection de défaut suivant l'une des revendications précédentes, dans lequel l'unité (31) d'application du courant est constituée de manière à imprimer un courant alternatif et/ou un courant continu sur les voies (6a ; 6b) de signal utile.

5. Dispositif de détection de défaut suivant l'une des revendications précédentes, dans lequel l'unité (31) d'application du courant est constituée de manière à appliquer individuellement les courants ($I_{E1}$ ; $I_{E2}$) imprimés respectivement sur une voie (6a ; 6b) de signal utile.

6. Dispositif de détection de défaut suivant l'une des revendications précédentes, dans lequel l'unité (31) d'application du courant est constituée de manière à ce que les signaux ($I_{E1}$ ; $I_{E2}$) imprimés sur les voies (6a ; 6b) de signal utile comprennent des courants ($I_{E1}$ ; $I_{E2}$) positifs.

7. Dispositif de détection de défaut suivant l'une des revendications précédentes, dans lequel l'unité (31) d'application du courant est constituée de manière à appliquer, à un certain nombre de voies de signal utile, un courant positif et, à un certain nombre de voies de signal utile, un courant négatif.

8. Dispositif de détection de défaut suivant l'une des revendications précédentes, dans lequel les signaux ($I_{E1}$ ; $I_{E2}$) imprimés se distinguent par voie (6a ; 6b) de signal utile d'au moins 5 nA et/ou au maximum de 20 nA.

9. Dispositif de détection de défaut suivant l'une des revendications précédentes, comprenant une deuxième voie (22) de signal parasite pour la mesure d'un deuxième signal ($I_{CM}$) parasite.

10. Dispositif de détection de défaut suivant la revendication 9, comprenant une unité (18) d'évaluation de signal parasite, dans lequel l'unité (18) d'évaluation de signal parasite est constituée de manière à former un signal ($I_k$) de combinaison, de préférence un signal ($I_k$) de différence du premier signal ($I_{RLD}$) parasite et du deuxième signal ($I_{CM}$) parasite.

11. Système (1) de mesure de tension, comprenant au moins un circuit (2) de mesure de signal comprenant un certain nombre de voies (6a ; 6b) de signal utile pour la mesure de signaux (BS) bioélectriques et comprenant un dispositif (40 ; 41 ; 42) de détection de défaut suivant l'une des revendications précédentes.

12. Système de mesure de tension suivant la revendication 11, comportant au moins deux voies (6a ; 6b) de signal utile.

13. Procédé de détection de défaillances (D) de voie de signal dans un système (1) de mesure de tension pour la mesure de signaux (BS) bioélectriques, dans lequel on mesure les signaux (BS) bioélectriques au moyen d'un circuit (2) de mesure de signal ayant un certain nombre de voies (6a ; 6b) de signal utile, dans lequel le procédé comprend au moins les stades suivants :

- impression d'au moins un premier signal ($I_{E1}$ ; $I_{E2}$) sur au moins une première voie (6a ; 6b) de signal utile au moyen d'au moins une première unité (31) d'application du courant, de manière à ce que le courant imprimé soit dans le domaine du nanoampère afin de ne pas fausser la mesure de signaux bioélectriques,

- contrôle, au moyen d'au moins une première unité (32) de comparaison, si le signal ($I_{E1}$ ; $I_{E2}$) de la première voie (6a ; 6b) de signal utile se trouve dans une plage de mesure en partant du fait que le signal passe sur la voie de signal utile,

- mesure par détection de courant d'au moins un premier signal ($I_{RLD}$) parasite sur au moins une première voie (7S) de signal parasite constituée en voie de mesure du courant,

- analyse, au moyen d'une unité (30) d'analyse de défaillance de voie de signal, afin de détecter une défaillance (D) de voie de signal dans une voie (6a ; 6b) de signal utile du système (1) de mesure de tension, si le signal ($I_{E1}$ ; $I_{E2}$) imprimé n'est pas mesuré sur la première voie (7S) de signal parasite et si le signal ($I_{E1}'$ ; $I_{E2}'$) contrôlé de l'unité (32) de comparaison est dans la plage de mesure.

**14.** Produit de programme d'ordinateur, comprenant un programme d'ordinateur, qui peut être chargé directement dans un dispositif de mémoire d'un système (1) de mesure de tension suivant la revendication 11, comprenant des parties de programme pour exécuter tous les stades du procédé suivant la revendication 13, lorsque le programme d'ordinateur est exécuté dans le système (1) de mesure de tension.

**15.** Support, déchiffrable par ordinateur, sur lequel est mis en mémoire un programme d'ordinateur suivant la revendication 14.

FIG 1

# FIG 2

# FIG 3

FIG 4

FIG 5

FIG 6

FIG 7 Stand der Technik

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5776133 A